# FASCICULE DE BREVET EUROPEEN

(11) **EP 2 953 541 B1**
(45) Date de publication et mention de la délivrance du brevet: **13.03.2019**
(21) Numéro de dépôt: 14705879.6
(22) Date de dépôt: 05.02.2014
(51) Int. Cl.: A61B 5/1455, A61B 5/1477, A61B 5/00

(54) **MÉTHODE NON INVASIVE POUR MESURER L'ÉTAT DE LA PERFUSION TISSULAIRE**
NICHTINVASIVES VERFAHREN ZUM MESSEN EINES GEWEBEDURCHBLUTUNGSZUSTANDS
NON-INVASIVE METHOD FOR MEASURING TISSUE PERFUSION STATE

(30) Priorité: 05.02.2013 FR 1350985
(43) Date de publication de la demande: 16.12.2015
(73) Titulaire: Assistance Publique Hôpitaux De Paris, 75004 Paris 4 (FR)
(72) Inventeur: VALLEE, Fabrice, F-93260 Les Lilas (FR); MATEO, Joaquim, F-75018 Paris (FR); PAYEN DE LA GARANDERIE, Didier, F-75018 Paris (FR)
(74) Mandataire: Gevers & Orès
(86) Numéro de dépôt international: PCT/IB2014/058815
(87) Numéro de publication internationale: WO 2014/122597

(56) Documents cités:
- WO-A1-2011/024018
- US-B1- 6 654 622
- MARTIN DRES ET AL: "Hemodynamic management of cardiovascular failure by using PCOvenous-arterial difference", JOURNAL OF CLINICAL MONITORING AND COMPUTING, KLUWER ACADEMIC PUBLISHERS, DO, vol. 26, no. 5, 25 juillet 2012 (2012-07-25), pages 367-374, XP035108484, ISSN: 1573-2614, DOI: 10.1007/S10877-012-9381-X

## Description

La présente invention concerne le domaine du diagnostic. Plus particulièrement, elle concerne une méthode non invasive pour déterminer l'état de la perfusion tissulaire chez un patient.

Les états de choc, quelle que soit leur origine (cardiogénique, hémorragique et/ou septique), entrainent une baisse de la perfusion tissulaire avec une atteinte plus ou moins marquée de la perfusion des petits vaisseaux. Cette altération microcirculatoire entraine une dysfonction voire une défaillance de différents organes, qui aggrave le pronostic des patients. Actuellement, il existe un besoin de moniteurs capables de refléter l'état de la perfusion microcirculatoire. Parmi les appareils récemment développés, certains moniteurs mesurent le CO₂ tissulaire et l'indice de perfusion (en anglais, *perfusion index*, ou *PI*).

La mesure de la pression partielle en CO₂ dans les tissus (PₜᵢₛₛᵤCO₂) a été identifiée comme un marqueur fiable de la perfusion tissulaire dans de nombreuses publications : une augmentation du CO₂ tissulaire, en particulier comparé au CO₂ artériel (calcul du gradient tissu - artère: Pₜ₋ₐCO₂), traduit une accumulation tissulaire du CO₂ du fait d'une baisse concomitante de la perfusion. Les dispositifs actuellement disponibles pour la mesure du CO₂ tissulaire sont la tonométrie gastrique (P_{g}CO₂) ou la mesure sublinguale (Pₛ₁CO₂). Récemment, la mesure du CO₂ cutané (P_{c}CO₂) au lobe de l'oreille a été décrite comme mesure du CO₂ tissulaire (WO 2011/024018). A l'instar des autres mesures de CO₂ tissulaire, une élévation du CO₂ cutané au lobe de l'oreille est un marqueur de mauvais pronostic dans les états de choc et un reflet fiable de la perfusion microcirculatoire.

L'indice de perfusion (PI), dérivé du signal de pléthysmographie de la saturation en oxygène (SpO₂), reflète la partie pulsatile du signal de SpO₂. Ce paramètre et surtout ses variations au cours de la prise en charge des patients ont été décrits comme un reflet fiable de la perfusion tissulaire.

Cependant, le diagnostic d'une altération de la perfusion microcirculatoire au cours d'un état de choc ne permet pas de guider la thérapeutique car ces altérations peuvent être de différentes étiologies et donc nécessiter des thérapeutiques très diverses. On peut rencontrer schématiquement deux situations:
(1) Problème "central prédominant" : dû à une hypoperfusion généralisée du fait d'un bas débit cardiaque entraînant une hypoperfusion macrocirculatoire et donc également une hypoperfusion microcirculatoire (situation rencontrée à la phase aigue d'un état de choc hémorragique ou cardiogénique). Dans cette situation, il n'y a le plus souvent pas d'atteinte de l'intégrité de la fonction endothéliale et microcirculatoire, l'hypoperfusion périphérique est due à une baisse de la perfusion globale. Après restauration de l'hémodynamique centrale, l'hypoperfusion microcirculatoire peut soit se normaliser rapidement, soit diminuer sans se normaliser totalement du fait de la vasonconstriction des territoires périphériques (comme la peau) privilégiant la perfusion des organes nobles. Ce cas correspond à un problème "distributif", du fait de l'anomalie de redistribution du débit sanguin central vers les débits régionaux, lequel entraîne une hypoperfusion périphérique résiduelle mais sans anomalie structurelle des microvaisseaux et de la fonction endothéliale.
(2) Problème "périphérique prédominant" : dû à une altération fonctionnelle de l'état des microvaisseaux avec raréfaction vasculaire et destruction de l'intégrité de l'endothélium vasculaire, entrainant une abolition de la vasodilatation réactionnelle et créant un véritable obstacle à la perfusion tissulaire sans possibilité adaptative. Dans ce cas, on n'observe pas, ou peu d'hypoperfusion centrale, mais une atteinte directe de la fonction microcirculatoire, entraînant une hypoperfusion périphérique. Cette situation se rencontre dans les cas de choc septique après prise en charge hémodynamique initiale, mais également dans des pathologies diverses où l'atteinte de la réactivité vasculaire et de la microcirculation est fréquente et influence le pronostic des patients (par exemple dans le cas de patients diabétiques, de patients hypertendus chronique, de patients porteurs de maladie de système avec angéite, de patients insuffisant rénaux, *etc*.).

Ces deux situations peuvent donner la même évaluation quantitative de l'altération de la perfusion microcirculatoire (même élévation du CO₂ tissulaire et même diminution du PI), alors qu'elles nécessitent des thérapeutiques foncièrement différentes. Dans la situation (1), dite "centrale prédominante", on tentera de restaurer rapidement et de manière durable l'hémodynamique centrale, ce qui entrainera une amélioration progressive de la perfusion microcirculatoire. Dans la situation (2), dite "périphérique prédominante", le pronostic est plus grave du fait de l'atteinte structurelle de la microcirculation ; le traitement étiologique est prépondérant et des thérapeutiques spécifiques à cette situation sont encore à l'étude actuellement.

La présente invention propose une méthode non-invasive qui permet de distinguer les deux situations décrites ci-dessus, et donc d'apporter au clinicien une information précieuse sur l'état de son patient. Cette méthode repose sur la mesure du CO₂ cutané et/ou du PI au cours d'une épreuve d'hyperthermie, pour tester l'intégrité de la microcirculation. Une "épreuve d'hyperthermie" se définit ici comme une augmentation locale et transitoire de la température d'une zone cutanée, de préférence une zone richement vascularisée. Par exemple, la peau de la zone choisie peut être portée à une température comprise entre 40 et 48°C, par exemple 45°C, pendant une durée de quelques minutes. Ceci permet de tester la capacité du réseau microcirculatoire à créer une vasodilatation induite par l'augmentation de la température. Schématiquement, cette vasodilatation réactionnelle à l'hyperthermie sera présente dans un profil type "central prédominant", témoignant d'une conservation des propriétés de l'endothélium vasculaire, et absente ou faible dans le profil "périphérique prédominant", du fait de la destruction ou l'altération fonctionnelle de ce dernier.

La présente invention porte donc, en premier lieu, sur une méthode non invasive pour tester la fonctionnalité de l'endothélium vasculaire au niveau du réseau microcirculatoire d'un patient, comportant les étapes suivantes :
(i) mesures en normothermie : à l'aide d'un ou plusieurs capteur(s) non chauffés ou maintenu(s) à une température de l'ordre de 37°C (choisie entre 20°C et 37°C, par exemple), on mesure la pression de CO₂ cutanée (P_{c}CO₂) et l'indice de perfusion (PI) du patient ;
(ii) épreuve d'hyperthermie : le ou les capteur(s) sont ensuite portés à une température choisie entre 40°C et 48°C, par exemple 45°C, et maintenus à cette température pendant une durée donnée de l'ordre de quelques minutes (par exemple comprise entre 3 et 15 minutes), puis ramenés à leur température initiale; au cours de cette épreuve, on mesure les variations de la pression de CO₂ cutanée et de l'indice de perfusion du patient;
(iii) interprétation :
   - une baisse de la pression de CO₂ cutanée au cours de l'épreuve d'hyperthermie, accompagnée d'une forte augmentation de l'indice de perfusion (augmentation supérieure à un seuil pré-déterminé), indique que l'endothélium vasculaire au niveau du réseau microcirculatoire du patient est fonctionnel ;
   - une augmentation de la pression de CO₂ cutanée au cours de l'épreuve d'hyperthermie, accompagnée d'une augmentation modérée (inférieure à un seuil pré-déterminé) de l'indice de perfusion, est indicative d'une atteinte du réseau microcirculatoire du patient.

Les variations de la pression de CO₂ cutanée sont, dans les exemples ci-dessous, étudiées en calculant la différence P_{c}CO_{2 fin} - P_{c}CO_{2début}, notée "P_{c}CO_{2 fin-début}". Bien entendu, cette variation peut être évaluée par tout autre calcul à partir des valeurs de la pression de CO₂ cutanée mesurée au cours du test, susceptible de donner une information sur la variation de P_{c}CO₂. Par exemple, on peut calculer le pourcentage de cette variation de la façon Suivante : (P_{c}CO_{2 fin} - P_{c}CO_{2début}) / (P_{c}CO_{2 fin} ou P_{c}CO_{2début}) x 100.

Selon une mise en oeuvre particulière de la méthode ci-dessus, illustrée dans les exemples, l'augmentation de l'indice de perfusion est calculée comme le ratio entre les valeurs maximale et minimale de cet indice au cours du test complet (incluant les mesures en normothermie). En pratique, cela revient à calculer le ratio entre la valeur maximale de l'indice de perfusion au cours de l'épreuve d'hyperthermie (PIₘₐₓ) et sa valeur en normothermie (PI_{37°C}). Ce ratio sera désigné dans ce qui suit "PIₘₐₓ/PIₘᵢₙ". Bien entendu, la variation de l'indice de perfusion peut être mesurée en effectuant un autre calcul, comme par exemple en calculant la différence PIₘₐₓ - PIₘᵢₙ, soit en valeur nette soit en pourcentage (PIₘₐₓ - PIₘᵢₙ)/(PIₘₐₓ ou PIₘᵢₙ) x 100, ou tout autre calcul à partir des valeurs de PI susceptible de donner une information sur la variation de l'indice de perfusion.

Comme illustré ci-dessous, le seuil pré-déterminé pour l'interprétation de l'augmentation de l'indice de perfusion est de préférence compris entre 2 et 4.

Dans une mise en oeuvre préférée de l'invention, un dispositif unique intègre le capteur de CO₂ et celui pour mesurer l'indice de perfusion. Ce dispositif sera appelé abusivement dans ce qui suit "le capteur".

Il est important de placer le capteur dans une zone richement vascularisée, afin d'obtenir des données fiables sur la microcirculation tissulaire. Dans une mise en oeuvre préférée de l'invention, la pression de CO₂ cutanée et l'indice de perfusion sont donc mesurés par un capteur placé au niveau du lobe d'une oreille.

Un exemple de matériel utilisable pour mettre en oeuvre la méthode décrite ci-dessus est l'électrode du TOSCA® 500 (Radiometer®, Copenhague) avec la technologie Masimo Set®. En effet, cette électrode permet de mesurer la pression de CO₂ cutanée en mm Hg (P_{c}CO₂) à 37°C, à condition de s'affranchir de la correction automatique, comme décrit dans l'article de Vallée *et al.* (Vallee *et al*., 2008) et dans la demande de brevet WO 2011/024018, et de mesurer l'indice de perfusion. En outre, l'électrode du TOSCA 500 possède un dispositif intégré qui permet de chauffer le capteur jusqu'à 45°C. Cette technologie a été développée pour créer une vasodilatation intense créant une "artérialisation" des tissus, rapprochant ainsi la valeur cutanée de PCO₂ (P_{c}CO₂) de la valeur artérielle (PₐCO₂), pour une surveillance des patients atteints de pathologies respiratoires.

Selon une mise en oeuvre préférée, l'épreuve d'hyperthermie est réalisée de la façon suivante : après stabilisation de la valeur de P_{c}CO₂ et du PI à 37°C, la température du capteur est augmentée à 45°C pendant 5 min puis redescendue à 37°C. Les variations de la valeur de P_{c}CO₂ et du PI sont analysées au cours de cette épreuve d'hyperthermie et interprétées comme décrit ci-dessus et illustrées dans la partie expérimentale.

Outre les variations de la P_{c}CO₂ et du PI, d'autres paramètres peuvent être mesurés lors de la mise en oeuvre de la méthode selon l'invention, et apporter des compléments d'information relatifs à l'état de la perfusion tissulaire du patient. En particulier, on peut mesurer le temps écoulé entre la fin de l'épreuve d'hyperthermie et le moment où le capteur recommence à consommer de l'énergie pour maintenir sa température à 37°C (c'est-à-dire entre 36,5 et 37,5°C). Ce temps, dit "temps de re-chauffage", est lié à la qualité de la perfusion tissulaire et au fait que la vasodilatation induite par le chauffage perdure après l'arrêt du chauffage. En effet, à température ambiante stable, la puissance du courant électrique nécessaire pour chauffer le capteur à une température constante est dépendant de la qualité de la perfusion tissulaire locale, principale source de chaleur pour le tissu cutané. Schématiquement, si la perfusion tissulaire est bonne, le moniteur n'a pas besoin de chauffer beaucoup le capteur pour conserver une température constante de 37°C, et inversement. L'homme du métier est en mesure, par des tests de routine, de calibrer ce paramètre en fonction des différentes conditions dans lesquelles le test est effectué (pièce, température ambiante *etc*.), pour interpréter ce paramètre.

Une variante du paramètre ci-dessus est l'énergie consommée par le capteur pour atteindre la température de 45°C ou pour maintenir sa température à 37°C pendant un laps de temps déterminé commençant à la fin de l'épreuve d'hyperthermie. De même que pour le paramètre précédent, si la perfusion tissulaire est bonne, le capteur consommera peu d'énergie pour atteindre la température de 45°C ou pour conserver une température constante de 37°C, et inversement. Ce paramètre peut également être calibré par des mesures de routine.

Le monitorage de la P_{c}CO₂ couplée au test de chauffage permet une évaluation et un suivi de l'état de la perfusion tissulaire. Le résultat du test et son évolution peuvent permettre de guider la thérapeutique dans différentes pathologies où la perfusion tissulaire est altérée. Une amélioration de la perfusion tissulaire sera notée par une baisse de la valeur de P_{c}CO₂ de base, et au chauffage une baisse de la valeur de P_{c}CO₂ au cours du test (baisse de P_{c}CO_{2 fin-début}) et une augmentation de la pulsatilité (augmentation de PIₘₐₓ/PIₘᵢₙ).

La méthode décrite ci-dessus, dans ses différentes variantes, est particulièrement utile dans les services de réanimation, pour déterminer l'état de la perfusion microcirculatoire d'un patient en état de choc, qu'il s'agisse d'un choc septique, hémorragique, allergique, cardiogénique ou autre.

De façon remarquable, cette méthode apporte une nouvelle information qui, dans le contexte d'un tableau clinique donné, aide le praticien hospitalier dans son pronostic pour ces patients, en particulier pour les patients en choc septique, et l'aide à mieux les prendre en charge. En effet, les inventeurs ont observé que si le test décrit ici montre que la perfusion microcirculatoire du patient est bonne, ce patient a une plus grande probabilité de survivre 28 jours après le début de son choc que dans le cas contraire.

Il est important de noter que la méthode décrite ici n'est pas limitée aux patients en état de choc. Au contraire, elle peut être utilisée pour aider au diagnostic, au pronostic et à la prise en charge de patients présentant des symptômes et pathologies divers, où l'atteinte de la réactivité vasculaire et de la microcirculation est fréquente et peut influencer le pronostic et/ou la prise en charge des patients. Pour les patients présentant de tels symptômes et/ou atteints de telles pathologies, la méthode présentée ici permet d'obtenir une information sur la gravité de l'atteinte de la perfusion tissulaire. Cette information s'ajoute au tableau clinique analysé par le praticien et constitue donc un élément supplémentaire pour établir un diagnostic et/ou un pronostic et/ou pour adapter le traitement du patient. A titre d'exemples non limitatifs des situations dans lesquelles la méthode de l'invention peut être avantageusement utilisée, on peut citer, outre les états de chocs cités plus haut, le diabète, l'artérite, dont l'artérite oblitérante des membres inférieurs (AOMI), l'hypertension, les maladies de système avec angéite, l'insuffisance rénale, *etc*. La méthode selon la présente invention est aussi utile pour connaître l'état de différents tissus après une greffe. A titre d'exemples non limitatifs illustrant cette application, on peut citer les lambeaux musculo-cutanés de reconstruction en chirurgie et la prise de greffe cutanée après brulure.

Selon les situations cliniques, le résultat du test décrit ici entraînera une prise en charge différente. Par exemple, pour un patient en choc septique, si le test décrit ici montre que la perfusion microcirculatoire du patient est faible (augmentation de la pression de CO₂ cutanée au cours de l'épreuve d'hyperthermie et/ou augmentation inférieure à un seuil prédéterminé de l'indice de perfusion), une dose de médicament vasoconstricteur sera administrée au patient. Parmi les médicaments utilisables dans cette situation, on peut citer la vasopressine et ses analogues, comme la terlipressine, la somatostatine et ses analogues, parmi lesquels l'octérotide, la dobutamine, l'épinéphrine et la norépinéphrine, appelée également lévartérénol ou noradrénaline.

A l'inverse, un résultat du test montrant que la perfusion microcirculatoire d'une greffe, par exemple d'un lambeau greffé, est faible, entraînera l'administration d'un traitement vasodilatateur et/ou antiagrégant et/ou anticoagulant. A titre exemples de traitement vasodilatateur administrable dans ce cas, on peut citer les dérivés nitrés (tels que la trinitrine, le dinitrate d'isosorbide, la molsidomine, le nicorandil, ...) et les analogues de la prostacycline (tel que l'Iloprost), lesquels peuvent être administrés localement, par exemple par voie percutanée (timbre) ou par injection, ou par voie générale (voie orale ou voie sublinguale). Le même type de traitement sera administré dans d'autres cas d'atteinte locale dans laquelle la microcirculation est diminuée, comme par exemple dans des cas d'artériopathie oblitérante d'un membre (souvent, un membre inférieur -AOMI). En complément ou à la place d'un tel traitement, le médecin pourra décider depratiquer une revascularisation par thombectomie chirurgicale ou thrombolyse *in situi.*

Différentes applications de la méthode selon la présente invention sont citées ci-dessous, à titre illustratif et non limitatif :

### 1- Au cours des états de chocs (septiques, cardiogéniques, hémorragiques, allergiques) :

- Introduction, suivi et sevrage d'un traitement vasoconstricteur type Norepinephrine, Epinephrine, Vasopressine, Dobutamine, *etc*. : prise de décision thérapeutique en fonction de l'effet sur la perfusion tissulaire.
- Monitorage de l'effet du remplissage vasculaire sur la perfusion tissulaire.

### 2- Au cours de la chirurgie vasculaire et la chirurgie reconstructrice avec lambeaux.

- Monitorage de la perfusion tissulaire avant et après pontage vasculaire, permettant de tester la viabilité et l'efficacité du pontage, et d'agir en conséquence (par administration de médicaments vasodilatateurs, anticoagulant et/ou antiagrégant, et/ou traitement chirurgical).
- Monitorage de la perfusion tissulaire avant et après revascularisation par la technique de Fogarty (thrombectomie chirurgicale) ou thrombolyse *in situ*, voire thromboaspiration, permettant de tester la viabilité et l'efficacité du geste de revascularisation.
- Monitorage de l'effet des médicaments anticoagulant type Héparine, pour ajustement de la dose administrée si nécessaire.
- Monitorage de l'effet des médicaments antiagrégant type Aspirine, antiGP2B3A, pour ajustement de la dose administrée si nécessaire.
- Monitorage de l'effet des médicaments vasodilatateurs type dérivés nitrés, analogues de la prostacycline (Iloprost), pour ajustement de la dose administrée si nécessaire.

### 3- Au cours de l'artériopathie oblitérante des membres inférieurs (AOMI) notamment chez le diabétique

- Monitorage de l'effet de l'introduction des médicaments anticoagulant type héparine ou AVK sur la perfusion tissulaire, pour ajustement du traitement si nécessaire.
- Monitorage de l'effet des médicaments antiagrégant type Aspirine, antiGP2B3A, pour ajustement de la dose administrée si nécessaire.
- Monitorage de l'effet des médicaments sur l'athérome type Statines, pour ajustement de la dose administrée si nécessaire.
- Monitorage de l'effet des médicaments vasodilatateurs type dérivés nitrés, analogues de la prostacyclines (Iloprost), inhibiteurs de l'enzyme de conversion (IEC), pour ajustement de la dose administrée ou du traitement si nécessaire.
- Monitorage de la perfusion tissulaire avant et après revascularisation par la technique de Fogarty (thrombectomie chirurgicale) ou thrombolyse *in situ*, voire thromboaspiration, permettant de tester la viabilité et l'efficacité du geste de revascularisation.
- Monitorage de l'effet d'un programme d'entrainement supervisé de la marche pour la réadaptation vasculaire des patients porteurs d'AOMI.

La présente invention porte également sur un dispositif pour effectuer un test de perfusion microcirculatoire tel que décrit plus haut. Un tel dispositif comprend :
- un capteur de mesure de la pression de CO₂ cutanée (P_{c}CO₂) ;
- un capteur de mesure de la saturation pulsée de l'hémoglobine en oxygène (SpO₂) ; de préférence, ces deux capteurs sont intégrés dans un seul et même objet, par exemple adapté au lobe de l'oreille.
- pour chaque capteur, des moyens de chauffage, de contrôle et de mesure de la température, capables de chauffer le capteur et maintenir sa température à une température choisie (entre 40°C et 48°C); la plage de température peut être plus ou moins étendue que la plage précitée. Elle est plus étendue si les moyens de chauffage sont aussi utilisés pour maintenir le capteur à une température choisie pour les mesures en normothermie (lesquelles peuvent être obtenues en maintenant le capteur à une température comprise entre la température ambiante et 37°C). Si les mesures en normothermie sont obtenues sans chauffer le capteur, la plage de 40 à 48°C peut au contraire être réduite, par exemple entre 44 et 46°C. L'homme du métier choisira, selon des critères économiques et l'étendue des applications qu'il souhaite donner au dispositif selon l'invention, des moyens de chauffage et de contrôle de la température appropriés.

- des moyens informatiques connectés aux deux capteurs, permettant de :
   (i) programmer le changement de température des capteurs pour la mise en oeuvre de la méthode (mesures en normothermie, épreuve d'hyperthermie puis retour à 37°C) ;
   (ii) afficher et/ou enregistrer les paramètres suivants au cours du test :
      - la pression de CO₂ cutanée (*N*.*b*. : ce paramètre doit être présenté sans correction par une formule intégrant la température du capteur, ou cette correction doit être annulée lors de l'étape de calcul de P_{c}CO_{2 fin - début}), et
      - l'indice de perfusion (PI) ;
   (iii) calculer la différence de pression de CO₂ cutanée entre la fin et le début de l'épreuve d'hyperthermie (P_{c}CO_{2 fin - début}), ainsi que le ratio entre les valeurs maximales et minimales de l'indice de perfusion au cours du test (PIₘₐₓ/PIₘᵢₙ).

Dans une réalisation préférée du dispositif selon l'invention, les moyens informatiques sont également capables d'afficher et/ou enregistrer la puissance délivrée par le capteur au cours du test, et/ou de calculer l'énergie consommée par le capteur pendant un laps de temps déterminé commençant à la fin de l'épreuve d'hyperthermie.

Les exemples et figures suivants illustrent l'invention sans toutefois limiter son étendue.
Figure 1 : *Flow chart* de l'étude préliminaire.
Figure 2 : Comparaison de la valeur de P_{c}CO₂ des patients choqués (noir) à l'inclusion des patients, par rapport à des contrôles sains (gris)
Figure 3 : Comparaison de la valeur de P_{c}CO_{2 fin-début} à l'inclusion des patients
Figure 4 : Evolution du gradient P_{c-Et}CO₂ des patients choqués, au cours des 48 heures suivant leur admission, en fonction de leur devenir
Figure 5 : Comparaison du ratio PIₘₐₓ/PIₘᵢₙ à l'inclusion des patients
Figure 6 : Evolution de P_{c}CO_{2 fin-début} au cours des premières 48h de réanimation en fonction du diagnostic des patients
Figure 7 : Evolution du rapport PIₘₐₓ/PIₘᵢₙ au cours des premières 48h de réanimation en fonction du diagnostic des patients
Figure 8 : Evolution P_{c}CO_{2 fin-début} au cours des premières 48h de réanimation en fonction du pronostic des patients (mortalité à J28)
Figure 9 : Evolution de PIₘₐₓ/PIₘᵢₙ au cours des premières 48h de réanimation en fonction du pronostic des patients (mortalité à J28)
Figure 10 : Schéma descriptif du test selon l'invention
Figure 11 : Données typiquement obtenues lors du test, pour un patient ne présentant pas d'atteinte fonctionnelle de l'endothélium vasculaire au niveau du réseau microcirculatoire (profil "central prédominant")
Figure 12 : Données typiquement obtenues lors du test, pour un patient présentant une atteinte fonctionnelle de l'endothélium vasculaire au niveau du réseau microcirculatoire (profil "périphérique prédominant")

### Exemples

### Exemple 1 : Etude préliminaire : variations de la pression de CO₂ cutanée (P_{c}-CO₂) et de l'index de perfusion (PI) lors d'une épreuve d'hyperthermie pour évaluer la microcirculation dans les états de choc

### 1.1. Matériels et méthodes

### Population étudiée

L'étude a été réalisée de décembre 2011 à septembre 2012 dans l'unité de réanimation chirurgicale de l'hôpital Lariboisière. Tous les patients en choc septique, cardiogénique ou hémorragique hospitalisés dans notre unité ont été inclus. Des patients de réanimation non choqués ont été analysés comme témoins ainsi que des volontaires sains. Il n'y a pas eu de modification de prise en charge thérapeutique en fonction des résultats obtenus des tests d'hyperthermie. Tous les patients ont été traités selon les habitudes courantes du service sans prendre en compte le résultat du test.

### Protocoles de mesures

Les mesures ont été réalisées après inclusion à moins de 24h du début du choc. A H0, H6, H12, H24, H36 et H48, toutes les données cliniques et biologiques ont été collectées et un test d'hyperthermie dynamique a été réalisé. Les paramètres collectés à chaque temps sont la température, les données hémodynamiques, les gaz du sang artériel et veineux (le sang veineux provenait d'un cathéter jugulaire interne), le lactate artériel, le débit cardiaque, le volume d'éjection systolique, le taux de CO₂ en fin d'expiration (EtCO₂). Chaque traitement ou mesure thérapeutique a été relevé.

### Mesures de la P_{c}CO₂

La P_{c}CO₂ (mm Hg) a été mesurée au lobe de l'oreille avec un moniteur TOSCA 500 (TOSCA®, Radiometer Basel Ag; Basel, Basel-Stadt, Switzerland) à 37°C sans constante métabolique (technique décrite dans la demande WO2011024018). Le capteur a été calibré *in vitro* avant utilisation puis systématiquement toutes les 12 heures. Puis le capteur a été fixé à un clip placé sur l'oreille du patient après application d'un gel contact. Appareil TOSCA 500, marquage CE n°0037 ; matériel référencé à l'APHP.

### Mesures de l'indice de perfusion

Le PI (%), paramètre mesurant la proportion de signal pulsée dans l'obtention du signal de SpO2, a été mesuré par la technologie Masimo (Masimo SET®). Cette technologie est intégrée à l'électrode du TOSCA 500.

### Test d'hyperthermie

Il a été réalisé à chaque temps : H0, H6, H12, H24, H36 et H48, quand la mesure de la P_{c}CO₂ à 37°C était stable. Le moniteur TOSCA 500 permet de régler la température de l'électrode de 37°C à 45°C par paliers de 0,5°C. Le test d'hyperthermie a consisté à chauffer l'électrode à 45° pendant cinq minutes puis à la remettre à 37°.

### Analyse statistique

Les paramètres du test de chauffage et leur évolution ont été comparés en fonction du diagnostic des patients et en fonction de leur devenir à J28 d'hospitalisation par ANOVA pour mesures répétées et test t de student.

### 1.2. Résultats

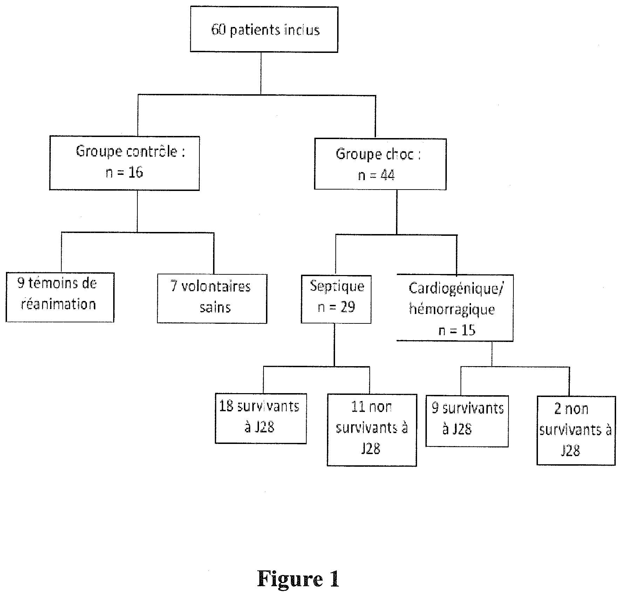

Au total, 60 patients ont été inclus, repartis en trois groupes (figure 1).

### Confirmation des résultats de l'étude décrite dans la demande WO2011/024018

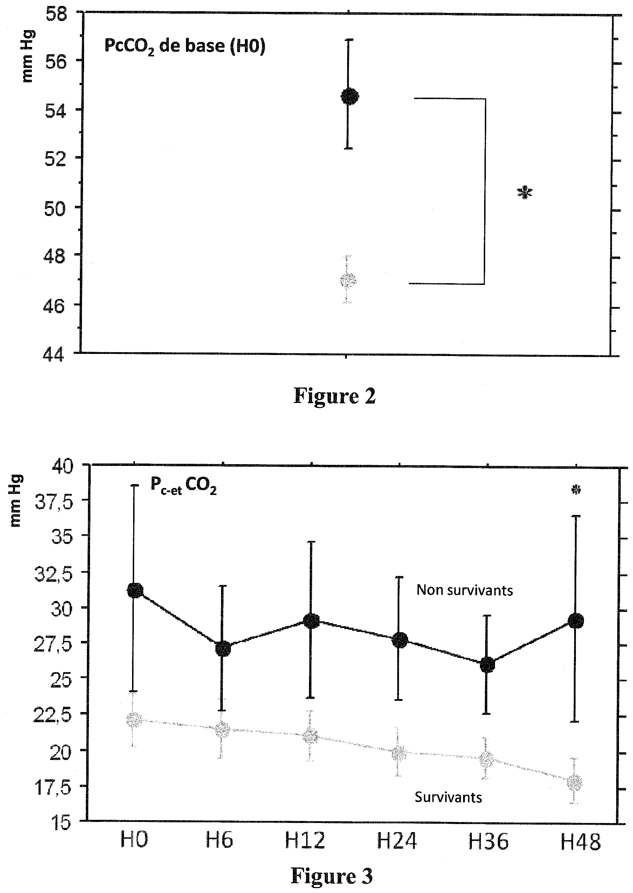

Les patients choqués ont une P_{c}CO₂ significativement plus élevée que les patients contrôles (Figure 2).

Au cours des 48 premières heures de réanimation, le gradient P_{c-et}CO₂ des patients qui ont finalement survécu (à 28 jours) a statistiquement baissé, contrairement à la situation observée pour les non survivants (Figure 3).

### Comparaison de la valeur de P_{c}CO_{2 fin-début} à l'inclusion des patients

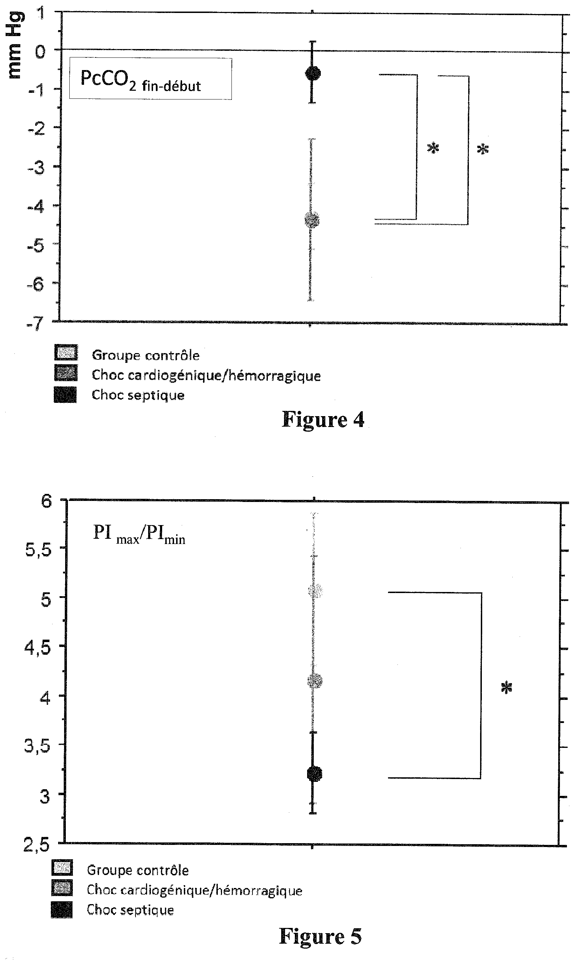

Les patients du groupe contrôle, ainsi que les patients du groupe choc cardiogénique/hémorragique baissent significativement plus leur P_{c}CO₂ au cours du test d'hyperthermie que les patients du groupe choc septique :
P_{c}CO_{2 fin-début}= -4.25 ± 3.28 *versus* -0.55 ± 4.31 mm Hg, p<0.01, et
P_{c}CO_{2 fin-début}= -4.33 ± 8.01 versus 0.55 ± 4.31 mm Hg, p=0.048, respectivement (Figure 4).

### Comparaison du ratio PIₘₐₓ/PIₘᵢₙ à l'inclusion des patients.

Au cours du test de chauffage, la valeur du PI est multipliée en moyenne par 5 dans le groupe contrôle, par 4 dans le groupe cardiogénique/hémorragique et par 3 dans le groupe septique (Figure 5). Cette différence est significative entre les contrôles et les patients septiques : 5.09 ± 3.09 versus 3.22 ± 2.18, p=0.02.

### Evolution de P_{c}CO_{2 fin-début} au cours des premières 48h de réanimation en fonction du diagnostic des patients

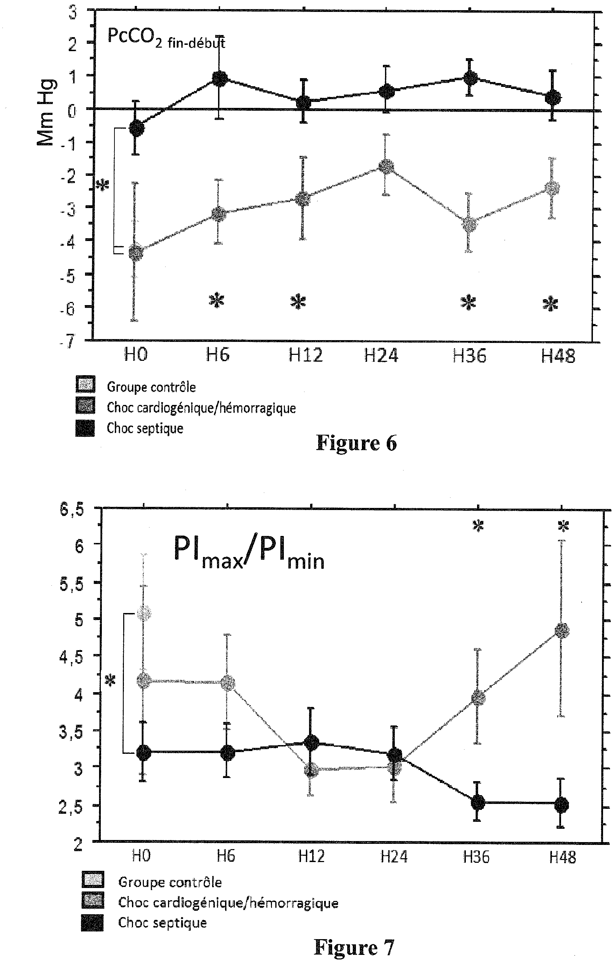

A partir de H6, les patients en choc septique augmentent leur P_{c}CO₂ au cours du test (P_{c}CO_{2 fin-début} positif), alors que les chocs cardiogénique/hémorragique continuent à baisser la P_{c}CO₂ (Figure 6). Cette différence est significative à H6, H12, H36 et H48.

### Evolution du rapport PIₘₐₓ/PIₘᵢₙ au cours des premières 48h de réanimation en fonction du diagnostic des patients

Dans l'évolution des premières 48h, on voit une tendance à l'augmentation du ratio PIₘₐₓ/PIₘᵢₙ dans le groupe cardiogénique/hémorragique, contrairement au groupe des chocs septiques (Figure 7). A H48, les patients du groupe cardiogénique/hémorragique retrouvent une valeur de PIₘₐₓ/PIₘᵢₙ proche de celle des patient contrôles à H0: 4.89 ± 4.07 et 5.09 ± 3.09. De plus, à H36 et à H48, la valeur du PIₘₐₓ/PIₘᵢₙ chez les patients du groupe cardiogénique/hémorragique devient significativement plus haute comparée aux patients septiques (3.97 ± 2.19 *versus* 2.56 ± 1.21, p=0.02 et 4.89 ± 4.07 *versus* 2.53 ± 1.34, p=0.03 respectivement).

### Evolution P_{c}CO_{2 fin-début} au cours des premières 48h de réanimation en fonction du pronostic des patients (mortalité à J28)

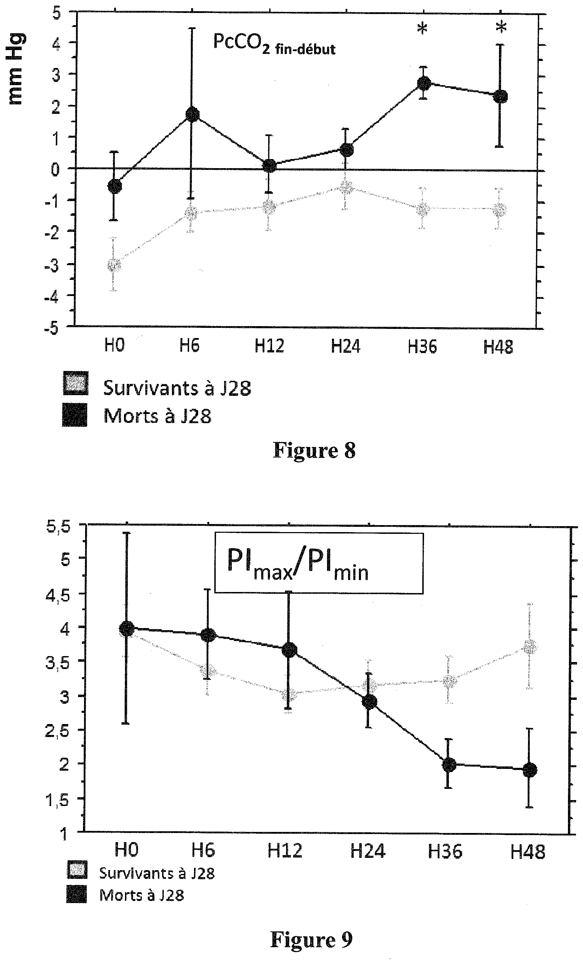

A partir de H6, les non survivants augmentent leur P_{c}CO₂ au cours de l'épreuve d'hyperthermie (P_{c}CO_{2 fin-début} positif), alors que les survivants la baissent systématiquement (P_{c}CO_{2 fin-début} négatif) au cours des 48 premières heures (Figure 8). Cette différence est significative à H36 et H48 :
P_{c}CO_{2 fin-début} = 2.80 ± 1.10 *vs.* -1.21 ± 3.34 mm Hg, p=0.01, et
P_{c}CO_{2 fin-début} = 2.40 ± 3.65 *vs.* -1.19 ± 3.16, p=0.03, respectivement.

### Evolution de PIₘₐₓ/PIₘᵢₙ au cours des premières 48h de réanimation en fonction du pronostic des patients (mortalité à J28)

Contrairement aux survivants, les non survivants baissent significativement leur ratio PIₘₐₓ/PIₘᵢₙ au cours des 36 premières heures : de 3.99 ± 5.05 à 1.97 ± 1.14, p=0.03 (Figure 9). Le PIₘₐₓ/PIₘᵢₙ des non survivants est significativement plus bas que les survivants à H48 : 1.97 ± 1.14 versus 3.76 ± 3.13, p=0.10. De plus, l'évolution du PIₘₐₓ/PIₘᵢₙ au cours des premières 48h est significativement différente entre les survivants et non-survivants p=0.02 (§ : p<0.05 avec ANOVA pour mesures répétées).

### 1.3. Discussion

Les résultats de cette étude confirment les résultats présentés dans la demande WO 2011/024018 :
1- Les patients en états de choc ont une P_{c}CO₂ à 37°C plus élevée que les patients contrôles.
2- Les survivants baissent leur gradient P_{c-Et}CO₂ dans les 48 premières heures comparativement aux non-survivants.

En outre, ces résultats montrent qu'au cours d'une épreuve d'hyperthermie :
1- Les patients sans atteinte fonctionnelle de la perfusion microcirculatoire (patients contrôles et les patients en choc cardiogénique ou hémorragiques) baissent leur CO₂ cutané (P_{c}CO_{2 fin-début} = - 4 mmHg en moyenne) à H0. Cette baisse est également retrouvée au cours des 48 premières heures pour les patients en choc cardiogénique ou hémorragique.
2- Les patients en choc cardiogénique ou hémorragique à 48h de prise en charge retrouvent une pulsatilité (PIₘₐₓ/PIₘᵢₙ) identique aux patients contrôles.
3- Les patients en choc septique, ayant une atteinte fonctionnelle plus grave de la perfusion microcirculatoire avec dysfonction endothéliale, ont des résultats au test d'hyperthermie différents puisqu'ils augmentent globalement leur P_{c}O₂ lors du test (P_{c}CO₂ fin-début positif) et ont une réponse pulsatile réduite à l'hyperthermie (PIₘₐₓ/PIₘᵢₙ bas).
4- Cette réponse au test d'hyperthermie est reliée au pronostic : dans les 48 premières heures, les patients qui vont survivre ont toujours une diminution de leur P_{c}CO₂ au cours de l'épreuve d'hyperthermie, avec une restauration progressive de la pulsatilité (augmentation du PIₘₐₓ/PIₘᵢₙ).

### 1.4. Conclusion

Le test décrit ici, avec épreuve d'hyperthermie en complément de l'analyse du CO₂ cutané à 37°C, permet de tester l'intégrité de la fonction endothéliale et microcirculatoire au cours des états de choc à savoir :
- P_{c}CO_{2 fin-début} négatif: le chauffage du capteur entraine une vasodilatation qui permet de d'éliminer le CO₂ produit par le tissu ;
- PIₘₐₓ/PIₘᵢₙ élevé (> 3) : le chauffage du capteur augmente la pulsatilité de la perfusion tissulaire.

Ces résultats montrent qu'une mauvaise réponse au test d'hyperthermie (P_{c}CO_{2 fin-début} positif et PIₘₐₓ/PIₘᵢₙ bas (<2)) confirme l'atteinte de la fonction endothéliale et microcirculatoire au cours de l'état de choc et influence le pronostic des patients.

### Exemple 2 : Test de perfusion microcirculatoire intégrant une épreuve d'hyperthermie

### 2.1. Description du test

La P_{c}CO₂ de base est la mesure de la P_{c}CO₂ avant de commencer l'épreuve d'hyperthermie, quand le capteur est à 37°.

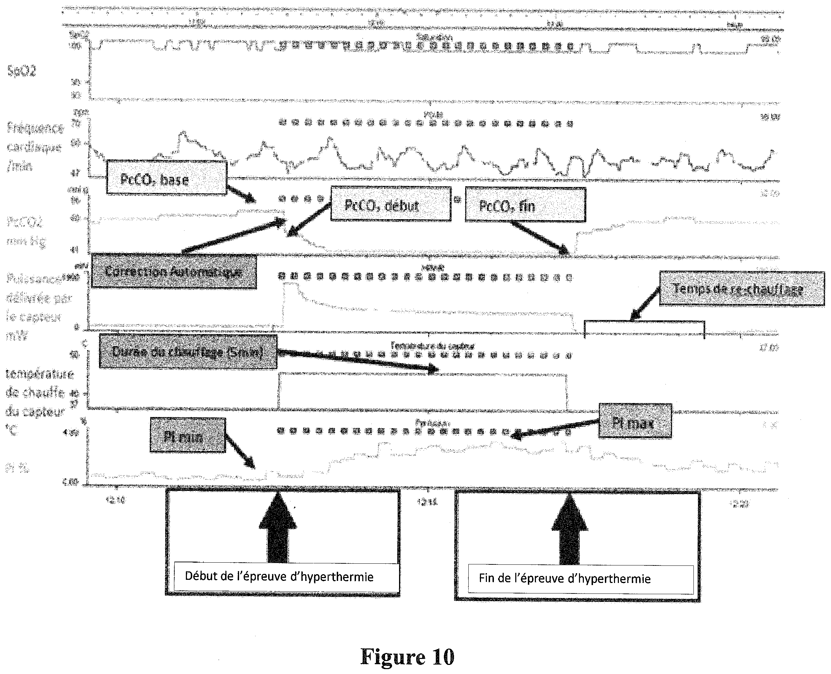

Les paramètres calculés avec les données du test sont illustrés à la figure 10. Il s'agit de :
- P_{c}CO_{2 fin-début} : c'est la différence entre la valeur de la P_{c}CO₂ au bout de cinq minutes de chauffage du capteur à 45° C et la valeur initiale à 37°C. En pratique, lorsque l'appareil utilisé effectue une correction automatique, on prend pour valeur initiale (P_{c}CO_{2 début}) la valeur de la P_{c}CO₂ immédiatement après le début du chauffage, lorsque la correction automatique a intégré le changement de température du capteur mais que le chauffage n'a pas encore eu d'impact sur la valeur de la pression cutanée de CO₂.

*N.b.* : la correction automatique abaisse systématiquement la valeur de la P_{c}CO₂ affichée en fonction de l'élévation de la température suivant la relation suivante : P_{c}CO₂affichée = P_{tc}CO₂ mesurée / 10^{(0.019x(T°-37)}, où T° est la température du capteur (Severinghaus et al., transcutaneous blood gas analysis. Respiratory Care, 1982, Hazinski and Severinghaus, 1982).
- PIₘₐₓ/PIₘᵢₙ est le ratio entre la valeur du PI la plus élevée (PIₘₐₓ) pendant l'épreuve d'hyperthermie et la valeur du PI la plus basse (PIₘᵢₙ) pendant l'ensemble du test (incluant la mesure avant l'épreuve d'hyperthermie et, dans certains cas, un laps de temps après l'arrêt de l'épreuve d'hyperthermie). En pratique, PIₘᵢₙ = PI à 37°C, avant le test de chauffage.
- Le "temps de re-chauffage" : ce paramètre correspond au temps, exprimé en secondes, à partir duquel le capteur se met à réchauffer la peau pour obtenir une température de 37°C après l'épreuve d'hyperthermie.

### 2.2. Test type : problème "central prédominant"

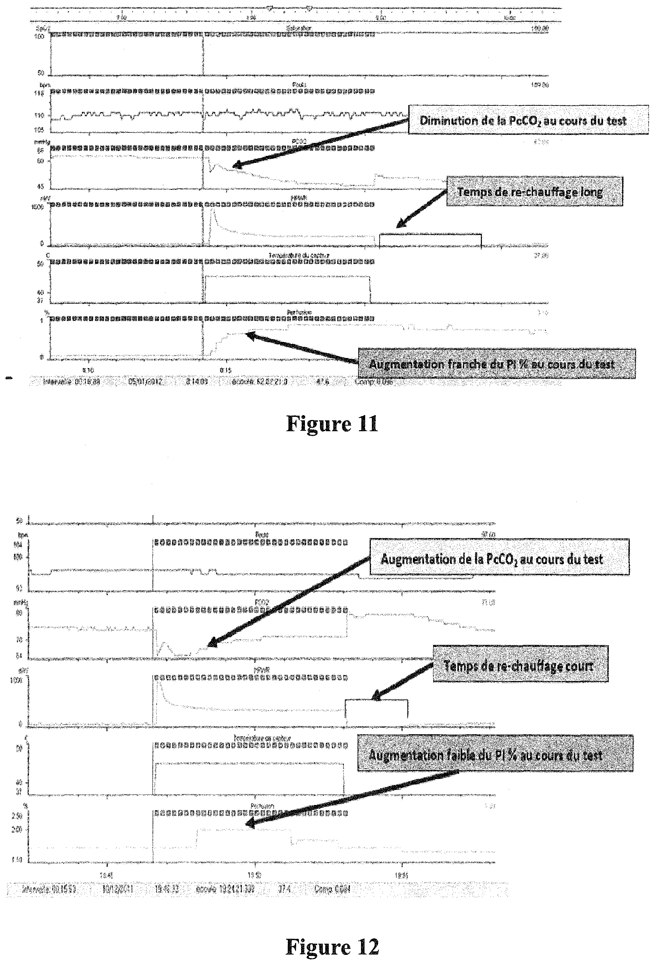

La figure 11 illustre les données typiquement obtenues lors du test selon l'invention, pour un patient ne présentant pas d'atteinte fonctionnelle de l'endothélium vasculaire au niveau du réseau microcirculatoire.

La vasodilatation induite par le chauffage a permis d'augmenter le débit de perfusion tissulaire et la pulsatilité, entrainant un rinçage du CO₂ produit par les tissus. Il n'y a pas d'atteinte fonctionnelle de la microcirculation, puisqu'il existe une vasodilatation induite par le chauffage. De plus, cette vasodilatation réactionnelle est durable puisque l'appareil n'a pas besoin de re-chauffer rapidement le capteur pour obtenir une température stable de 37°C. Il s'agit donc d'un profil "central prédominant". La thérapeutique consistera en une restauration et un maintien de l'hémodynamique centrale et une surveillance de la normalisation de la perfusion tissulaire périphérique avec normalisation de la valeur de la P_{c}CO₂ et du PI.

### 2.3. Test type : problème "périphérique prédominant"

La valeur de P_{c}CO₂ augmente (P_{c}CO_{2 fin-début} positif), le PI ne change pas ou peu (PImax/PImin <2), et le temps de re-chauffage du capteur est court: Il s'agit certainement d'un profil "périphérique prédominant". Le CO₂ produit par le chauffage à 45°C n'est pas rincé et s'accumule dans le tissu. Le chauffage n'entraîne pas (ou peu) de vasodilatation ni d'augmentation de la pulsatilité et de la perfusion microcirculatoire. Le moniteur est obligé de re-chauffer rapidement après la fin du test pour maintenir une température constante du capteur. Cela démontre l'altération fonctionnelle de la microcirculation avec dysfonction des propriétés de l'endothélium vasculaire. Le pronostic est plus grave que dans le cas précédent.

### 2.4. Conclusion

L'épreuve d'hyperthermie se positionne donc en complément de l'analyse de la P_{c}CO₂ en normothermie au lobe de l'oreille (demande WO 2011/024018) : l'augmentation de la P_{c}CO₂ révèle l'anomalie de la perfusion microcirculatoire et le test d'hyperthermie, couplant une analyse de la P_{c}CO₂, du PI et, le cas échéant, du temps de re-chauffage, permet de classifier cette anomalie et de tester l'intégrité du réseau microcirculatoire. Ce test non invasif et sans inconfort pour le patient, teste la fonctionnalité de l'endothélium vasculaire en monitorant l'importance de la vasodilatation induite par l'augmentation de la température. Il renseigne sur la gravité de l'atteinte microcirculatoire et peut permettre d'orienter la thérapeutique.

### Exemple 3 : Exemples d'évolution de patients selon la pathologie et le pronostic

### 3.1. Patient témoin de réanimation

Le test d'un patient témoin de réanimation montre que celui-ci n'a pas d'anomalie de la microcirculation. La valeur de P_{c}CO₂ diminue de -5 mm Hg au cours de l'épreuve d'hyperthermie. Le rapport PIₘₐₓ/PIₘᵢₙ à 7.5 montre lui aussi une réactivité préservée de la microcirculation, avec augmentation de la pulsatilité. Le temps de re-chauffage est supérieur à 200 secondes (Tableau 1).

**Tableau 1**

| | **H0** |
|---|---|
| P_{c}CO_{2 fin-début} | -5 |
| PIₘₐₓ/PIₘᵢₙ | 7,5 |
| Temps de re-chauffage(s) | 222 |

### 3.2. Patient en choc hémorragique de bon pronostic

Le test de chauffage d'un patient en choc hémorragique en cours de réanimation montre qu'il existe une réactivité microcirculatoire puisqu'il existe une diminution de la P_{c}CO_{2 fin-début} au cours du temps, avec un rapport PIₘₐₓ/PIₘᵢₙ toujours supérieur à 4 et un temps de re-chauffage long qui s'allonge au cours du temps (Tableau 2).

**Tableau 2**

| | **H0** | **H6** | **H12** | **H24** | **H36** | **H48** |
|---|---|---|---|---|---|---|
| P_{c}CO_{2 fin-début} | -14 | -4 | -6 | -6 | -5 | -4 |
| PIₘₐₓ/PIₘᵢₙ | 6 | 4,2 | 7 | 9 | 6 | 5,8 |
| Temps de re-chauffage(s) | 127 | 120 | 153 | 192 | 109 | 176 |

### 3.3. Patient en choc cardiogénique de bon pronostic

Chez ce patient en choc cardiogénique, l'épreuve d'hyperthermie montre qu'il existe une bonne réactivité vasculaire, ce qui entraîne un rinçage efficace du CO₂ tissulaire, d'où une valeur de P_{c}CO_{2 fin-début} toujours négative. La valeur du PIₘₐₓ/PIₘᵢₙ est elle toujours élevée et le temps de re-chauffage augmente au cours du temps (Tableau 3).

**Tableau 3**

| | H0 | H6 | H12 | H24 | H36 | H48 |
|---|---|---|---|---|---|---|
| P_{c}CO_{2 fin-début} | -22 | -14 | -12 | -3 | -7 | -8 |
| PIₘₐₓ/PIₘᵢₙ | 5 | 2,3 | 5 | 5 | 8 | 13 |
| Temps de re-chauffage(s) | 130 | 152 | 229 | 207 | 216 | 214 |

### 3.4. Patient septique de bon pronostic

Chez ce patient en choc septique, l'épreuve d'hyperthermie montre qu'il existe une réactivité vasculaire conservée qui permet de faire diminuer la valeur P_{c}CO_{2 fin-début} et de faire augmenter le rapport PIₘₐₓ/PIₘᵢₙ. Le temps de re-chauffage est lui aussi relativement long. Il n'y a donc pas d'atteinte fonctionnelle de la microcirculation (Tableau 4).

**Tableau 4**

| | H0 | H6 | H12 | H24 | H36 | H48 |
|---|---|---|---|---|---|---|
| P_{c}CO_{2 fin-début} | 0 | -5 | -6 | -2 | -4 | -3 |
| PIₘₐₓ/PIₘᵢₙ | 2,3 | 4 | 2,4 | 6,25 | 4,2 | 3,6 |
| Temps de re-chauffage(s) | 247 | 207 | 208 | 169 | 179 | 193 |

### 3.5. Patient septique de mauvais pronostic

Il s'agit d'un patient en choc septique sur péritonite qui est décédé à J1 de défaillance multivicérale. Au cours des épreuves d'hyperthermie, la valeur de la P_{c}CO₂ augmente (P_{c}CO₂ fin-début positif), la valeur du PI n'augmente que faiblement (PImax/PImin < 2) et le temps de re-chauffage est très court (Tableau 5).

**Tableau 5**

| | H0 | H6 | H12 | H24 |
|---|---|---|---|---|
| P_{c}CO_{2 fin-début} | +6 | 0 | +3 | +4 |
| PIₘₐₓ/PIₘᵢₙ | 1,5 | 1,3 | 1,1 | 1,1 |
| Temps de re-chauffage (s) | 103 | 74 | 78 | 91 |

### 3.6. Patient après chirurgie reconstructrice

Patient de 42 ans opéré pour une tumeur ORL avec reconstruction par un lambeau libre de grand dorsal. Monitorage continu de la P_{c}CO2 et du PI sur le lambeau durant les trois premiers jours post opératoires. Réalisation du test de chauffage toutes les 12 heures et surveillance clinique de la viabilité du lambeau.

Dans ce type de chirurgie, la viabilité d'un lambeau libre est une question majeure pour le chirurgien en post opératoire. Le monitorage de la P_{c}CO2 avec test de chauffage peut renseigner sur la perfusion du lambeau et sa viabilité.

Résultat : on remarque une baisse progressive des valeurs statiques de P_{c}CO2 et, au cours des tests de chauffage, une baisse plus importante au cours du temps de la valeur de P_{c}CO2 au cours de l'épreuve d'hyperthermie (P_{c}CO2 _{fin-début} passant de 0 à -5mmHg de H0 à H48) et une augmentation de la pulsatilité au chauffage (PI _{max/min} de 0 à +5). Cela démontre une amélioration de la perfusion tissulaire du lambeau, ce qui a été confirmé cliniquement par la bonne viabilité du lambeau à moyen terme.

**Tableau 6**

| | H0 | H12 | H24 | H36 | H48 | H60 | H72 |
|---|---|---|---|---|---|---|---|
| P_{c}CO2 mm Hg | 72 | 68 | 60 | 55 | 52 | 50 | 50 |
| PI% | 0 | 0.2 | 0.3 | 0.2 | 0.1 | 0.3 | 0.6 |
| P_{c}CO2 _{fin-début} mmHg | 0 | 0 | -1 | -5.2 | -5.4 | -4.7 | -4.9 |
| PI _{max/min} | 0 | 2.2 | 3.3 | 3.5 | 4.1 | 4.2 | 5.6 |

Conclusion : cet exemple montre le grand intérêt de la mesure de la P_{c}CO2 et des tests de chauffage dans le cadre de la chirurgie avec pontage vasculaire. La mesure permet également d'optimiser les traitements vasoactifs utilisés dans ces situations (médicaments vasodilatateurs, tels que les dérivés nitrés)

### 3.7. Artériopathie de membres inférieurs chez le patient artéritique et diabétique

Patient de 65 ans, hypertendu diabétique, admis en réanimation dans les suites d'une chirurgie abdominale de la vésicule. A son arrivée, on remarque une asymétrie de coloration des jambes : la jambe droite est plus pâle, avec faible pouls périphérique comparativement à la jambe gauche.

La mesure comparative de la P_{c}CO2 avec test de chauffage a été effectuée dans ce cas clinique pour vérifier que le test de l'invention peut renseigner sur l'atteinte vasculaire des patients artéritiques.

Résultat : une asymétrie des valeurs statiques de P_{c}CO2 a été observée, ainsi qu'une mauvaise réactivité au chauffage de la jambe droite. Un Angio-Scanner a confirmé le diagnostic d'ischémie aigue du membre inférieur droit, et le patient a bénéficié d'une revascularisation par technique de Fogarty (embolectomie à la sonde de Fogarty). En post opératoire, la valeur de P_{c}CO2 est redescendue à des valeurs normales au niveau de la jambe droite avec une bonne réactivité au chauffage.

**Tableau 7**

| | Jambe gauche Pré-Op | Jambe gauche Post-Op | Jambe droite Pré-Op | Jambe droite Post-Op |
|---|---|---|---|---|
| P_{c}CO2 mm Hg | 48 | 47 | 88 | 55 |
| PI % | 1.4 | 1.8 | 0 | 0.8 |
| P_{c}CO2 _{fin-début} mmHg | -3.8 | -4.1 | +3 | -5 |
| PI _{max/min} | 4.2 | 3.7 | 0 | 4.2 |

Conclusion : cet exemple montre le grand intérêt de la mesure de la P_{c}CO2 et des tests de chauffage chez le patient artéritique, notamment diabétique, pour surveiller la vascularisation des membres inférieurs. La mesure de la P_{c}CO2 et le test de chauffage permettent également d'optimiser les traitements vasoactifs utilisés dans ces situations.

### REFERENCES

Severinghaus et al., transcutaneous blood gas analysis. Respiratory Care, 1982.
Hazinski, T.A. and Severinghaus, J.W. (1982) Transcutaneous analysis of arterial PCO2. Med Instrum, 16, 150-153.
Vallée, F., Vallet, B., Mathe, O., Parraguette, J., Mari, A., Silva, S., Samii, K., Fourcade, O. and Genestal, M. (2008) Central venous-to-arterial carbon dioxide difference: an additional target for goal-directed therapy in septic shock? Intensive Care Med, 34, 2218-2225.
Vallée F, Mateo J, Dubreuil G, Poussant T, Tachon G, Ouanounou I, Payen D. (2010) Cutaneous ear lobe PCO2 at 37°C to evaluate microperfusion in patients with septic shock.Chest. 2010 Nov;138(5):1062-70. doi: 10.1378/chest.09-2690. Epub 2010 May 14.
Dres M., Monnet X., Tebould J-L. (2012) Hemodynamic management of cardiovascular failure by using PCO2 venous-arterial difference. J Clin Monit Comput (2012) 26:367-374.

## Revendications

1. Méthode non invasive pour déterminer l'état de la perfusion microcirculatoire d'un patient, comportant les étapes suivantes :
(i) mesures en normothermie : à l'aide d'un ou plusieurs capteur(s) non chauffé(s) ou maintenu(s) à une température choisie entre 30°C et 37°C, effectuer les mesures suivantes :
• mesure de la pression de CO₂ cutanée (P_{c}CO₂) du patient ;
• mesure de l'indice de perfusion (PI) du patient ;
(ii) épreuve d'hyperthermie : augmenter la température du ou des capteur(s) à une température choisie entre 40°C et 48°C ; maintenir cette température pendant une durée comprise entre 3 et 15 minutes, puis ramener la température du ou des capteur(s) à la température initiale; mesurer les variations de la pression de CO₂ cutanée et de l'indice de perfusion du patient au cours de cette épreuve ;
(iii) interprétation :
• une baisse de la pression de CO₂ cutanée au cours de l'épreuve d'hyperthermie, accompagnée d'une augmentation de l'indice de perfusion supérieure à un seuil pré-déterminé, indique que l'endothélium vasculaire au niveau du réseau microcirculatoire du patient est fonctionnel ;
• une augmentation de la pression de CO₂ cutanée au cours de l'épreuve d'hyperthermie, accompagnée d'une augmentation de l'indice de perfusion inférieure à un seuil pré-déterminé, est indicative d'une atteinte du réseau microcirculatoire du patient.

2. Méthode selon la revendication 1, dans laquelle l'augmentation de l'indice de perfusion est calculée comme le ratio de la valeur maximale de cet indice au cours de l'épreuve d'hyperthermie et de la valeur de cet indice en normothermie (PIₘₐₓ/PIₘᵢₙ).

3. Méthode selon la revendication 2, dans laquelle le seuil pré-déterminé pour l'interprétation de l'augmentation de l'indice de perfusion est compris entre 2 et 4.

4. Méthode selon l'une quelconque des revendications 1 à 3, dans laquelle la pression de CO₂ cutanée et l'indice de perfusion sont mesurés par un capteur placé au niveau du lobe d'une oreille.

5. Méthode selon l'une quelconque des revendications précédentes, comprenant en outre la mesure du temps écoulé entre la fin de l'épreuve d'hyperthermie et le moment où le capteur recommence à consommer de l'énergie pour maintenir sa température à 37°C.

6. Méthode selon l'une quelconque des revendications précédentes, comprenant en outre la mesure de l'énergie consommée par le capteur pour en augmenter la température jusqu'à la température choisie pour l'épreuve d'hyperthermie, ou pour maintenir sa température à 37°C, pendant un laps de temps déterminé commençant à la fin de l'épreuve d'hyperthermie.

7. Méthode selon l'une quelconque des revendications précédentes, pour déterminer l'état de la perfusion microcirculatoire d'un patient en état de choc.

8. Méthode pour obtenir une information relative au pronostic des chances de survie à 28 jours d'un patient en état de choc, comprenant les étapes suivantes :
(i) déterminer l'état de la perfusion microcirculatoire du patient, par la méthode selon l'une quelconque des revendications précédentes ;
(ii) interprétation : si le résultat de l'étape (i) montre que l'endothélium vasculaire au niveau du réseau microcirculatoire est fonctionnel, l'information est en faveur d'un bon pronostic pour ce patient ; dans le cas contraire, elle suggère un mauvais pronostic.

9. Méthode selon la revendication 7 ou la revendication 8, **caractérisée en ce que** le patient est en état de choc septique.

10. Méthode selon l'une quelconque des revendications 1 à 6, pour déterminer l'état de la perfusion microcirculatoire d'un patient atteint d'une maladie où l'atteinte de la réactivité vasculaire et de la microcirculation influence le pronostic et la prise en charge.

11. Méthode selon la revendication 10, **caractérisée en ce que** patient est diabétique et/ou artéritique et/ou hypertendu et/ou porteur de maladie de système avec angéite, et/ou insuffisant rénal.

12. Dispositif pour effectuer un test de perfusion microcirculatoire en mettant en oeuvre la méthode selon l'une quelconque des revendications précédentes, **caractérisé en ce qu'**il comprend :
• un capteur de mesure de la pression de CO₂ cutanée (P_{c}CO₂) du patient ;
• un capteur de mesure de la saturation pulsée de l'hémoglobine en oxygène (SpO₂) ;
• pour chaque capteur, des moyens de chauffage et de mesure de la température, capables de chauffer les capteurs et maintenir leur température à une température choisie entre 40°C et 48°C ;
• des moyens informatiques connectés aux deux capteurs, permettant de :
(i) programmer le changement de température des capteurs pour la mise en oeuvre de la méthode ;
(ii) afficher et/ou enregistrer les paramètres suivants au cours du test :
- la pression de CO₂ cutanée,
- l'indice de perfusion (PI),
(iii) calculer la différence de pression de CO₂ cutanée entre la fin et le début de l'épreuve d'hyperthermie (P_{c}CO_{2 fin - début}), ainsi que le ratio entre les valeurs maximales et minimales de l'indice de perfusion au cours du test (PI_{max/}PIₘᵢₙ).

13. Dispositif selon la revendication 12, dans lequel les deux capteurs sont combinés en un seul et même objet.

14. Dispositif selon la revendication 12 ou la revendication 13, **caractérisé en ce que** les moyens informatiques sont configurés pour afficher et/ou enregistrer la puissance délivrée par le(s) capteur(s) au cours du test.

15. Dispositif selon l'une quelconque des revendications 12 à 14, **caractérisé en ce que** les moyens informatiques sont configurés pour calculer l'énergie consommée par le capteur pendant un laps de temps déterminé commençant à la fin de l'épreuve d'hyperthermie.

## Patentansprüche

1. Nichtinvasives Verfahren zum Bestimmen des mikrozirkulatorischen Perfusionszustands eines Patienten, aufweisend die folgenden Schritte:
(i) Normothermie-Messungen: mittels eines oder mehrerer Sensors/en, der/die nicht geheizt ist/sind oder auf einer ausgewählten Temperatur zwischen 30 °C und 37 °C gehalten wird/werden, Durchführen der folgenden Messungen:
- Messung des CO₂-Hautdrucks (P_{c}CO₂) des Patienten;
- Messung des Perfusionsindex (PI) des Patienten;
(ii) Hyperthermie-Prüfung: Erhöhen der Temperatur des oder der Sensors/en auf eine ausgewählte Temperatur zwischen 40 °C und 48 °C; Halten dieser Temperatur während einer Dauer zwischen 3 und 15 Minuten, dann Zurückbringen der Temperatur des oder der Sensors/en auf die Ausgangstemperatur; Messen der Variationen des CO₂-Hautdrucks und des Perfusionsindex des Patienten im Laufe dieser Prüfung;
(iii) Auswertung:
- ein CO₂-Hautdruckabfall im Laufe der Hyperthermie-Prüfung, begleitet von einer Erhöhung des Perfusionsindex, der höher als ein vorbestimmter Schwellenwert ist, weist darauf hin, dass das vaskuläre Endothel im Bereich des mikrozirkulatorischen Netzwerks des Patienten funktionell ist;
- eine CO₂-Hautdruckerhöhung im Laufe der Hyperthermie-Prüfung, begleitet von einer Erhöhung des Perfusionsindex, der niedriger als ein vorbestimmter Schwellenwert ist, ist für eine Beeinträchtigung des mikrozirkulatorischen Netzwerkes des Patienten hinweisend.

2. Verfahren nach Anspruch 1, wobei die Erhöhung des Perfusionsindex wie das Verhältnis des Maximalwertes dieses Index im Laufe der Hyperthermie-Prüfung und des Wertes dieses Normothermie-Index (PIₘₐₓ / PIₘᵢₙ) berechnet wird.

3. Verfahren nach Anspruch 2, wobei der vorbestimmte Schwellenwert für die Auswertung der Erhöhung des Perfusionsindex zwischen 2 und 4 liegt.

4. Verfahren nach einem der Ansprüche 1 bis 3, wobei der CO₂-Hautdruck und der Perfusionsindex durch einen Sensor gemessen werden, der im Bereich des Ohrläppchens eines Ohres platziert ist.

5. Verfahren nach einem der vorstehenden Ansprüche, weiter umfassend die Messung der Zeit, die zwischen dem Ende der Hyperthermie-Prüfung und dem Moment, in dem der Sensor erneut anfängt, Energie zu verbrauchen, um seine Temperatur auf 37 °C zu halten, vergangen ist.

6. Verfahren nach einem der vorstehenden Ansprüche, weiter umfassend die Messung der Energie, die vom Sensor verbraucht wurde, um seine Temperatur bis auf die für die Hyperthermie-Prüfung ausgewählte Temperatur zu erhöhen oder um seine Temperatur auf 37 °C zu halten, während eines bestimmten Zeitraums, der am Ende der Hyperthermie-Prüfung anfängt.

7. Verfahren nach einem der vorstehenden Ansprüche, um den mikrozirkulatorischen Perfusionszustand eines unter Schock stehenden Patienten zu bestimmen.

8. Verfahren, um eine Information bezüglich der Prognose der Überlebenschancen von 28 Tagen eines unter Schock stehenden Patienten zu erhalten, umfassend die folgenden Schritte:
(i) Bestimmen des mikrozirkulatorischen Perfusionszustands des Patienten, durch das Verfahren nach einem der vorstehenden Ansprüche;
(ii) Auswertung: wenn das Ergebnis des Schritts (i) zeigt, dass das vaskuläre Endothel im Bereich des mikrozirkulatorischen Netzwerkes funktionell ist, ist die Information zugunsten einer guten Prognose für diesen Patienten; im gegenteiligen Fall legt sie eine schlechte Prognose nahe.

9. Verfahren nach Anspruch 7 oder Anspruch 8, **dadurch gekennzeichnet, dass** der Patient unter septischem Schock steht.

10. Verfahren nach einem der Ansprüche 1 bis 6, um den mikrozirkulatorischen Perfusionszustand eines Patienten zu bestimmen, der an einer Erkrankung, in der die Beeinträchtigung der vaskulären Reaktivität und der Mikrozirkulation die Prognose und die Betreuung beeinflussen, leidet.

11. Verfahren nach Anspruch 10, **dadurch gekennzeichnet, dass** der Patient diabetisch und/oder arteriitisch und/oder hypertonisch und/oder Träger einer Systemerkrankung mit Angiitis, und/oder niereninsuffizient ist.

12. Vorrichtung, um einen mikrozirkulatorischen Perfusionstest durchzuführen, indem das Verfahren nach einem der vorstehenden Ansprüche umgesetzt wird, **dadurch gekennzeichnet, dass** sie umfasst:
- einen Sensor zur Messung des CO₂-Hautdrucks (P_{c}CO₂) des Patienten;
- einen Sensor zur Messung der pulsatilen Sauerstoffsättigung des Hämoglobins (SpO₂);
- für jeden Sensor, Mittel zur Erwärmung und zur Messung der Temperatur, die imstande sind, die Sensoren zu erwärmen und ihre Temperatur auf einer ausgewählten Temperatur zwischen 40 °C und 48 °C zu halten;
- Informatikmittel, die mit den zwei Sensoren verbunden sind, die es ermöglichen:
(i) die Temperaturänderung der Sensoren für die Umsetzung des Verfahrens zu programmieren;
(ii) die folgenden Parameter im Laufe des Tests anzuzeigen und/oder zu speichern:
- den CO₂-Hautdruck,
- den Perfusionsindex (PI),
(iii) den CO₂-Hautdruckunterschied zwischen dem Ende und dem Beginn der Hyperthermie-Prüfung (P_{c}CO_{2 fin - début}), sowie das Verhältnis zwischen den Maximal- und Minimalwerten des Perfusionsindex im Laufe des Tests (PIₘₐₓ / PIₘᵢₙ) zu berechnen.

13. Vorrichtung nach Anspruch 12, wobei die zwei Sensoren in ein und demselben Objekt kombiniert sind.

14. Vorrichtung nach Anspruch 12 oder Anspruch 13, **dadurch gekennzeichnet, dass** die Informatikmittel konfiguriert sind, um die durch den/die Sensor/en gelieferte Leistung im Laufe des Tests anzuzeigen und/oder zu speichern.

15. Vorrichtung nach einem der Ansprüche 12 bis 14, **dadurch gekennzeichnet, dass** die Informatikmittel konfiguriert sind, um die vom Sensor verbrauchte Energie während eines bestimmten Zeitraums, der am Ende der Hyperthermie-Prüfung anfängt, zu berechnen.

## Claims

1. Non-invasive method for determining the microcirculatory perfusion state of a patient, comprising the following steps:
(i) measurements in normothermia: using one or more sensors that are not heated or that are maintained at a temperature selected between 30°C and 37°C, taking the following measurements:
• measurement of the skin CO₂ pressure (P_{c}CO₂) of the patient;
• measurement of the perfusion index (PI) of the patient;
(ii) hyperthermia test: increasing the temperature of the sensor or sensors to a temperature selected between 40°C and 48°C; maintaining this temperature for a duration between 3 and 15 minutes, then bringing the temperature of the sensor or sensors to the initial temperature; measuring the variations in the skin CO₂ pressure and in the perfusion index of the patient during this test;
(iii) interpretation:
• a drop in the skin CO₂ pressure during the hyperthermia test, accompanied by an increase in the perfusion index greater than a predetermined threshold, indicates that the vascular endothelium at the level of the microcirculatory network of the patient is functional;
• an increase in the skin CO₂ pressure during the hyperthermia test, accompanied by an increase in the perfusion index less than a predetermined threshold, indicates that the microcirculatory network of the patient is afflicted.

2. Method according to claim 1, wherein the increase in the perfusion index is calculated as the ratio of the maximum value of this index during the hyperthermia test and of the value of this index in normothermia (PIₘₐₓ/PIₘᵢₙ).

3. Method according to claim 2, wherein the predetermined threshold for the interpretation of the increase in the perfusion index is between 2 and 4.

4. Method according to any of claims 1 to 3, wherein the skin CO₂ pressure and the perfusion index are measured by a sensor placed at the level of the lobe of an ear.

5. Method according to any of the preceding claims, further comprising the measurement of the time elapsed between the end of the hyperthermia test and the moment when the sensor starts again to consume energy for maintaining the temperature thereof at 37°C.

6. Method according to any of the preceding claims, further comprising the measurement of the energy consumed by the sensor in order to increase the temperature thereof until the temperature selected for the hyperthermia test, or to maintain the temperature thereof at 37°C, for a determined lapse of time starting at the end of the hyperthermia test.

7. Method according to any of the preceding claims, for determining the microcirculatory perfusion state of a patient in a state of shock.

8. Method for obtaining information relating to the survival prognosis at 28 days of a patient in a state of shock, comprising the following steps:
(i) determining the state of the microcirculatory perfusion of the patient, by the method according to any of the preceding claims;
(ii) interpretation: if the result of the step (i) shows that the vascular endothelium at the level of the microcirculatory network is functional, the information is in favour of a good prognosis for this patient; in the opposite case, it suggests a bad prognosis.

9. Method according to claim 7 or claim 8, **characterised in that** the patient is in a state of septic shock.

10. Method according to any of claims 1 to 6, for determining the microcirculatory perfusion state of a patient suffering from a disease where the affliction of the vascular reactivity and of the microcirculation influences the prognosis and the care.

11. Method according to claim 10, **characterised in that** patient is diabetic and/or arteritic and/or hypertensive and/or carrier of systemic disease with angiitis, and/or renal insufficiency.

12. Device for conducting a microcirculatory perfusion test by implementing the method according to any of the preceding claims, **characterised in that** it comprises:
• a sensor for measuring the skin CO₂ pressure (P_{c}CO₂) of the patient;
• a sensor for measuring pulsed haemoglobin oxygen saturation (SpO₂);
• for each sensor, means for heating and for measuring the temperature, capable of heating the sensors and maintaining their temperature at a temperature selected between 40°C and 48°C;
• computerised means connected to the two sensors, making it possible to:
(i) program the change in temperature of the sensors for the implementation of the method;
(ii) display and/or record the following parameters during the test:
- the skin CO₂ pressure,
- the perfusion index (PI),
(iii) calculating the difference in skin CO₂ pressure between the end and the beginning of the hyperthermia test (P_{c}CO_{2 fin - debut}), as well as the ratio between the maximum and minimum values of the perfusion index during the test (PIₘₐₓ/PIₘᵢₙ).

13. Device according to claim 12, wherein the two sensors are combined into one single and same object.

14. Device according to claim 12 or claim 13, **characterised in that** the computerised means are configured to display and/or record the power delivered by the sensor or sensors during the test.

15. Device according to any of claims 12 to 14, **characterised in that** the computerised means are configured to calculate the energy consumed by the sensor during a determined lapse of time starting at the end of the hyperthermia test.
